# DEMANDE DE BREVET EUROPEEN

(11) **EP 2 682 077 A1**
(43) Date de publication de la demande: **08.01.2014**
(21) Numéro de dépôt: 13290130.7
(22) Date de dépôt: 10.06.2013
(51) Int. Cl.: A61F 9/007

(54) **Sonde à chanfrein**

(30) Priorité: 06.07.2012 FR 1201918
(71) Demandeur: France Chirurgie Instrumentation SAS-FCI, 75015 Paris (FR)
(72) Inventeur: Urion, Stéphane, 70190 Cromary (FR)
(74) Mandataire: Eidelsberg, Olivier Nathan

(57) **Abrégé**

Sonde d'intubation comportant au moins une partie en forme de tube (9) en un matériau souple biocompatible, notamment en silicone, destinée à être solidarisée à un fil (7) de guidage, dont une extrémité (18), pour solidariser mutuellement la sonde et le fil, est introduite dans le tube par une ouverture (22) de ce dernier, caractérisée en ce que le tube comporte un tronçon (24), du côté de l'ouverture (22) du tube par laquelle l'extrémité du fil est introduite dans le tube, ayant une forme en biseau, le diamètre extérieur du tube, le long du tronçon (24), augmentant depuis l'ouverture (22).

## Description

La présente invention se rapporte à un système d'intubation, notamment monocanaliculaire ou bicanaliculaire, dit de Ritleng, qui comporte une canule d'intubation, un fil de guidage et une sonde, notamment en forme de tube, en particulier en silicone, cette dernière étant destinée à être insérée dans le canal lacrymal. La présente invention se rapporte aussi à un ensemble d'intubation constitué d'une sonde tube en matériau souple et d'un fil en matériau souple solidarisé à la sonde tube, ainsi qu'à une sonde tube de ce genre.

L'objectif est d'introduire la sonde en forme de tube en silicone dans les voies lacrymales. Pour se faire, on procède d'abord au cathétérisme des voies lacrymales du patient à l'aide d'une canule ouverte à une extrémité, fermée à l'extrémité opposée et comportant une fente s'étendant le long d'une génératrice de l'extrémité ouverte vers l'extrémité fermé, jusqu'à une lumière formée dans la paroi latérale de la canule, lumière qui a une plus grande dimension en largeur (mesurée perpendiculairement à l'axe longitudinal de la canule) que la fente. Ensuite, un fil souple est solidarisé au tube en silicone pour former un ensemble d'intubation et le fil est introduit à l'intérieur de la canule et est poussé hors de celle ci à travers la dite lumière jusque dans la fosse nasale. Le fil, classiquement en prolène, est alors récupéré dans la fosse nasale et on retire la canule des voies lacrymales en la faisant coulisser sur le fil et en la désolidarisant au niveau d'un tronçon aminci de ce dernier en faisant sortir ce tronçon aminci par la fente formée sur la longueur de la canule. Une fois la canule retirée, on tire sur le fil en prolène par le côté de la fosse nasale pour faire entrer la sonde tube en silicone, solidarisée à l'autre extrémité du fil, dans les voies lacrymales. Un système de Ritleng de ce genre est décrit dans le brevet français FR 2704749.

Bien que ce système de Ritleng ait présenté une avancée certaine pour l'introduction de la sonde tube de silicone dans les voies lacrymales, notamment par comparaison avec des systèmes tels que décrits dans US 6117116 qui sont de structures compliqués et entraînent un traumatisme assez sanglant lors de la mise en place du tube sonde dans le canal lacrymal, on souhaite améliorer encore plus cette introduction, notamment la rendre encore moins traumatique pour les voies lacrymales et augmenter le taux de réussite d'insertion convenable de la sonde tube en silicone dans les voies lacrymales.

La présente invention vise à surmonter les inconvénients de l'art antérieur en proposant une ensemble d'intubation de Ritleng destinée à être insérée dans les voies lacrymales par un fil de guidage solidarisé à la sonde pour la tirer en position dans le canal lacrymal dont l'insertion est moins traumatique pour le patient et qui présente un taux de réussite d'insertion plus élevé que dans l'art antérieur.

Suivant l'invention, un ensemble d'intubation est tel que défini à la revendication 1, des perfectionnements étant définis aux sous revendications 2 à 9.

De préférence, l'épaisseur du tube augmente depuis l'ouverture du tube le long du tronçon en biseau puis est constante après cela.

En prévoyant ainsi une forme en biseau de la partie en forme de tube de la sonde qui vient à la suite du fil solidarisé à la sonde, on s'assure qu'au passage entre le fil et le tube en silicone la transition se fait plus en douceur et par conséquent lorsqu'on tire la sonde par le fil pour la faire passer dans les voies lacrymales, le tube, grâce à cette forme en biseau, pénètre plus facilement en suivant plus facilement le fil guide, notamment sans que le bord du tube ne se retourne sur lui-même, et ce en étant moins traumatique pour les parois du canal lacrymal. Suivant l'invention, et contrairement aux sondes de l'art antérieur (voir par exemple celles décrites dans FR 2704749 ou US 6117116), la surface extérieure de la sonde tube, c'est à dire la surface de la sonde destinées à venir en contact avec la ou les parois internes du canal lacrymal en glissant contre celle ci lors de l'insertion de la sonde dans le canal, ne comporte pas d'arête vive susceptible de s'ancrer ou de se bloquer dans la paroi, ce qui entraînerait un repliement ou retournement de la sonde sur elle même ou des lésions ou traumatismes sur la paroi du canal. En outre, la structure de l'ensemble est simple, constituée simplement du fil et de la sonde, sans nécessiter de pièce de raccordement supplémentaire source d'une part de complication pour la fabrication et d'autre part de rigidité de l'ensemble au niveau du raccord, rendant l'ensemble moins facile à manipuler par le chirurgien lors de l'insertion.

La présente invention se rapporte également à une sonde d'intubation destinée à être solidarisée à un fil de guidage pour former un ensemble suivant l'invention ainsi qu'à un système d'intubation comportant un ensemble d'intubation suivant l'invention et une canule d'intubation, la canule étant insérée avant l'introduction de l'ensemble d'intubation de Ritleng dans le canal lacrymal.

A titre d'exemple, on décrit maintenant un mode de réalisation de l'invention en se rapportant aux dessins dans lesquels :
- la Figure 1: est une vue de dessus d'une canule d'un système d'intubation suivant l'invention ;
- la Figure 2: représente un ensemble d'intubation formant sonde de Ritleng suivant l'invention, dans lequel on voit bien la liaison entre le tube de silicone et le fil de guidage ;
- la Figure 3: est une vue schématique décrivant le procédé d'introduction du tube de silicone dans le canal lacrymal ;
- la Figure 4: représente un autre mode de réalisation d'une canule d'un système d'intubation suivant l'invention, vu en coupe longitudinale ; et
- la Figure 5: est une vue de dessus de la canule de la figure 4.

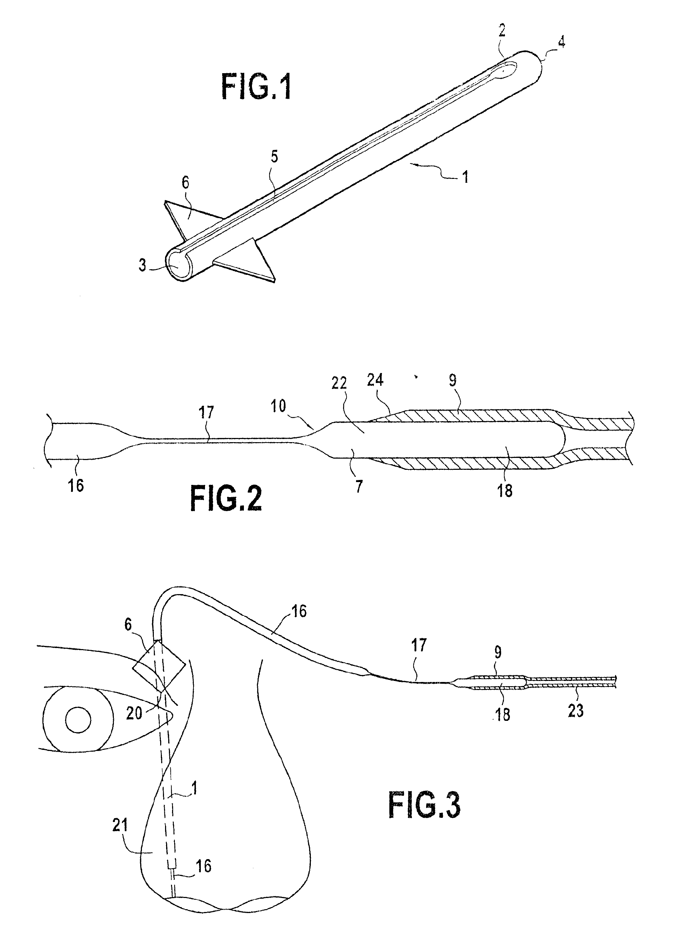

A la Figure 1, il est représenté une canule d'un système suivant l'invention. Cette canule 1 est constituée d'un corps cylindrique circulaire creux en matière semi rigide, par exemple en acier inoxydable ou en matière thermoplastique, notamment en PEEK ou en polyarylamide, ouvert à une extrémité 3 proximale et fermée à l'extrémité 4 opposée distale.

La canule 1 comporte une fente 5 longitudinale qui s'étend le long d'une génératrice du cylindre, de l'ouverture 3 proximale ouverte en direction de l'extrémité 4 fermée, jusqu'à une lumière 2 de plus grande largeur (mesurée perpendiculairement à l'axe longitudinal du cylindre, qui est parallèle à la fente longitudinale) que celle de la fente 5.

La lumière 2 a, vue de dessus comme à la figure 1, une forme en ellipse dont le petit diamètre est perpendiculaire à l'axe longitudinal et le grand diamètre parallèle à l'axe de la fente. Le petit diamètre est plus grand que l'épaisseur de l'interstice de la fente.

Des ailettes 6 de préhension font saillie latéralement de la canule, au voisinage de l'ouverture 3 proximale.

A la Figure 2, il est représenté en partie un ensemble 10 d'intubation dit de Ritleng. Cet ensemble 10 d'intubation dit de Ritleng est constitué d'un fil 7, notamment en prolène ou en un autre matériau biocompatible et souple analogue, et d'une sonde tube 9 en silicone.

Le fil comporte trois tronçons, à savoir un tronçon 16 d'extrémité de grand diamètre suivi d'un tronçon 17 intermédiaire de plus petit diamètre, lui-même suivi d'un tronçon 18 d'extrémité opposé de grand diamètre. Le diamètre du tronçon 16 de grand diamètre est tel qu'il peut passer dans le tube et dans la lumière 2 mais pas dans la fente 5. Le diamètre du tronçon 17 intermédiaire de plus petit diamètre est tel qu'il peut passer dans la fente 5.

La sonde 9 est constituée d'un tube formé par un cylindre circulaire creux dont la paroi a une épaisseur comprise de préférence entre 0,15mm et 0,5mm. Elle est notamment réalisée en silicone ou autre matériau analogue.

Le tronçon 18 d'extrémité opposé de grand diamètre est inséré dans le tube 9 en silicone pour les solidariser mutuellement. Cette solidarisation peut être effectuée par une insertion à force ou à adaptation serrée. On peut également les solidariser par collage ou tout autre procédé analogue, par exemple soudure à ultrason.

L'insertion de la sonde tube 9 dans le canal lacrymal s'effectue de la manière suivante :

On insère d'abord la canule 1 dans le canal lacrymal en y pénétrant par l'entrée 20 extérieure du côté de l'oeil jusqu'à la sortie 21 débouchant dans la fosse nasale.

Une fois la canule insérée, on y fait coulisser le tronçon 16 jusqu'à ce que l'extrémité du tronçon ressorte de la canule par la lumière 2. Le chirurgien saisit alors cette extrémité ressortie du tronçon 16 et en la tenant par exemple avec une pince adaptée (non représentée) il tire par les ailettes la canule 1 hors du canal lacrymal en Pour insérer le tube 9 sonde dans le canal lacrymal, on insère le tronçon 18 d'extrémité opposé de grand diamètre dans la canule et en prenant cette dernière par les ailettes 4, on insère l'ensemble (canule et fil) dans le canal lacrymal à partir de l'extérieur du côté de l'oeil jusqu'à ce qu'une extrémité du fil passant par la lumière 2 de la canule ressorte par l'intérieur des narines. On retire alors la canule 1 en tirant par les ailettes 4. La canule glisse le long du fil jusqu'à atteindre le tronçon 17 intermédiaire qui peut alors être passé par la fente 5 de la canule pour libérer cette dernière de sa coopération avec le fil. Une fois la canule 1 retirée, on tire le fil par son extrémité ressortant du canal lacrymal du côté de la narine jusqu'à ce que le tube 9 en silicone, solidaire du tronçon 18 du fil, vienne s'insérer dans le canal lacrymal. Une fois le tube 9 en silicone inséré dans le canal lacrymal, on coupe le fil de guidage qui pend à l'extérieur des narines.

La sonde 9 est ici constituée d'un tube ouvert des deux côtés. Du côté de l'ouverture 22 par laquelle le tronçon 16 est introduit dans le tube 9 pour les solidariser mutuellement, la paroi latérale 23 définissant le tube a une épaisseur qui varie de sorte qu'un tronçon (24) de bord du tube 9 ait une forme en biseau.

Lors de la mise en place du tube 9 en silicone par tirage du fil de guidage, le fait que le bord d'attaque du tube 9 de guidage ait une forme en biseau permet d'aider à la mise en place du tube 9 en silicone en faisant en sorte qu'il n'y ait pas, comme dans l'art antérieur, de bord d'attaque en arête vive qui vienne "taper" contre la paroi du canal lacrymal évitant ainsi soit d'endommager cette paroi du canal lacrymal et/ou soit un retournement du bord du tube 9 pouvant entraîner une désolidarisation du tronçon 16 d'avec le tube 9 avant d'avoir atteint la position finale souhaitée de ce dernier dans le canal lacrymal et donc un mauvais positionnement du tube 9 en silicone nécessitant d'aller le rechercher, puis de recommencer toute l'opération avec un nouveau tube en silicone. Ainsi, suivant l'invention, grâce au biseau ou chanfrein, l'insertion est moins traumatique vis-à-vis de la paroi du canal lacrymal et a un plus grand taux de réussite.

La surface extérieure de la sonde 9, c'est à dire la surface destinée à venir en contact avec la paroi du canal lacrymal lors de son insertion dans ce canal, notamment en glissant contre cette paroi, ne comporte pas d'arête vive, cette surface extérieure comportant un tronçon 24 d'extrémité incliné. Le tronçon 24 s'étend jusqu'à son extrémité libre de sorte que lorsque le fil 22 est inséré dans le tube, il n'y a pas non plus d'arête vive formée entre le fil et le tronçon 24 au niveau de l'extrémité libre de ce dernier, la transition entre la partie du fil 22 à l'extérieur du tube 9 et le tronçon se faisant en douceur, sans aspérité ou arête vive susceptible de se coincer ou de s'ancrer dans la paroi du canal.

Lors de l'insertion, les surfaces extérieures du fil et de la sonde au voisinage de l'ouverture 22 d'interface entre le fil et la sonde forment l'extérieur de l'ensemble et viennent en contact directement avec la paroi du canal lacrymal.

La souplesse (ou la rigidité, inverse de la souplesse) de l'ensemble est telle que en tout point de sa longueur, et notamment au niveau de la liaison fil-sonde, l'ensemble peut se déformer, par exemple se courber pour épouser la forme d'une courbe quelconque ou se plier pour former un angle et épouser la forme d'un croisement de canaux. En particulier, en tout point, l'ensemble est suffisamment souple pour pouvoir prendre une forme courbe et la conserver de soi même, sans revenir à une position droite, à la manière d'un fil.

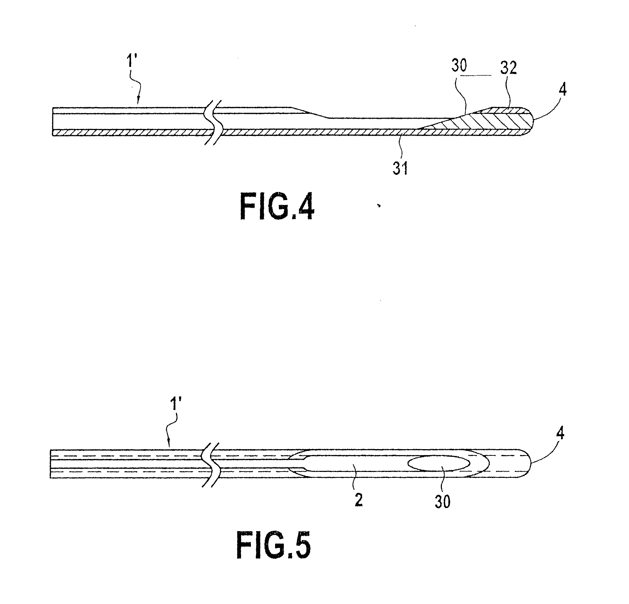

Aux figures 4 et 5, il est représenté une canule 1' suivant un autre mode de réalisation qui peut également être utilisée dans le système d'intubation décrit ci dessus à la place de la canule 1. Les parties identiques des deux canules 1 et 1' sont représentées par les mêmes références numériques.

Au delà de la lumière 2 et jusqu'à l'extrémité 4 distale opposée, la canule est pleine de sorte que le fil de guidage, lors de son insertion dans la canule, ne peut pas pénétrer dans la canule au delà de la lumière 2, venant buter contre la surface 30 intérieure de fond de la canule. Cette surface 30 intérieure est en biseau, en étant inclinée d'environ 18° par rapport à l'axe longitudinal de la canule.

La surface 30 intérieure formant le fond de la canule est plane. En coupe longitudinale, comme représenté à la figure 4, elle a la forme d'une droite qui s'étend d'un point 31 inférieur de la paroi intérieure latérale de la canule du côté opposé à la fente jusqu'à un point 32 supérieur de la paroi latérale de la canule du côté de la fente, le point 32 supérieur étant plus proche de l'extrémité 4 distale de la canule que le point 31. A la place d'une forme plane, on pourrait cependant prévoir une forme légèrement incurvée, concave ou convexe. La fonction de cette surface 30 formant le fond intérieur de la canule est de guider l'extrémité du fil de guidage pour le faire sortir par la lumière 2 lors de l'étape d'insertion du fil dans la canule, et l'empêcher ainsi de pénétrer dans une région de la canule au delà de la lumière 2, entre celle ci et l'extrémité distale, pour éviter, comme dans l'art antérieur, au fil d'y être coincé et obliger le chirurgien à aller rechercher ce fil bloqué dans l'espace au delà de la lumière.

Suivant un autre mode de réalisation non représenté, on peut prévoir non pas de remplir l'espace au delà de la lumière 2 mais de le fermer par une paroi qui constitue alors une surface de butée et de guidage pour le fil de guidage ayant la même fonction que la surface 30.

## Revendications

1. Ensemble d'intubation comportant :
- une sonde d'intubation destinée à être positionnée dans le canal lacrymal, comportant au moins une partie en forme de tube (9) en un matériau souple biocompatible, notamment en silicone ; et
- un fil (7), en un matériau souple et solidarisé à la sonde, par lequel la sonde est tirée pour être transportée en position dans le canal lacrymal ;
dans lequel un tronçon d'extrémité (16) du fil s'étend à l'intérieur de la sonde tube en passant par une ouverture (22) de cette dernière,
**caractérisé en ce que** la transition entre le fil et la sonde tube se fait en continuité, c'est à dire sans la présence d'arête susceptible de se coincer dans la paroi lacrymale lors du déplacement de la sonde tirée par le fil.

2. Ensemble suivant la revendication 1, **caractérisé en ce que** à partir de l'ouverture (22), la sonde tube comporte un tronçon en biseau, le diamètre extérieur du tube augmentant de l'ouverture (22) d'une valeur égale sensiblement au diamètre du fil.

3. Ensemble suivant la revendication 2, **caractérisé en ce que** après le tronçon en biseau, la sonde tube se poursuit en la forme d'un tube cylindrique.

4. Ensemble suivant l'une des revendications 1 à 3, **caractérisé en ce que** l'épaisseur du tube est sensiblement nulle au niveau de l'ouverture (22), puis augmente le long du tronçon en biseau, de sorte que lorsque le fil est introduit dans le tube, la transition entre le fil et le tube se fait en douceur, sensiblement de manière continue, sans présence d'une aspérité ou d'une arête vive.

5. Ensemble suivant l'une des revendications 1 à 4, **caractérisé en ce que** le fil est solidarisé au tube par adaptation serrée de ce dernier autour du fil.

6. Ensemble suivant l'une des revendications 1 à 5, **caractérisé en ce que** le fil est solidarisé au tube par collage.

7. Ensemble suivant l'une des revendications précédentes, **caractérisé en ce que** l'ensemble n'est constitué que du fil et du tube sonde, notamment sans pièce de raccordement au niveau de la liaison entre le fil et la sonde.

8. Ensemble suivant l'une des revendications précédentes, **caractérisé en ce que** la surface extérieure de l'ensemble au niveau de l'ouverture (22) n'est pas recouverte.

9. Ensemble suivant l'une des revendications précédentes, **caractérisé en ce qu'**en tout point, l'ensemble est suffisamment souple pour pouvoir prendre une forme courbe et la conserver de soi même, sans revenir à une position droite, à la manière d'un fil.

10. Système d'intubation comportant un ensemble d'intubation suivant l'une des revendications 1 à 9 et une canule d'intubation, la canule étant destinée à être insérée dans le canal lacrymal avant l'introduction de l'ensemble d'intubation.

11. Sonde tube destinée à faire partie d'un ensemble tel que défini à l'une des revendications 1 à 9.
